# EUROPEAN PATENT APPLICATION

(11) **EP 3 960 213 A1**
(43) Date of publication of application: **02.03.2022**
(21) Application number: 20193696.0
(22) Date of filing: 31.08.2020
(51) Int. Cl.: A61L 9/20, A61L 9/22, B01D 53/32, B01D 53/72, F24F 7/003, F24F 8/192, B03C 3/12, B03C 3/41, B03C 3/47

(54) **UV BASED DISINFECTION DEVICE**

(71) Applicant: Alpina Grills NV, 8560 Wevelgem (BE)
(72) Inventor: Surmont, Bart, 8560 Wevelgem (BE)
(74) Representative: BiiP cvba

(57) **Abstract**

An air disinfection device (1) comprising a housing defining a pathway having an air input and an air output and at least a first UV-based disinfection zone (2) and a second UV-based disinfection zone (3), both provided in serial configuration in the pathway between the air input and the air output, characterised in that at least a first (4) and a second (5) airstream modulator are provided in the pathway, the first airstream modulator (4) arranged upstream the first disinfection zone (2) and the second airstream modulator (4) arranged between the first and second UV-based disinfection zones (2, 3).

## Description

### Field of the Invention

The present invention concerns an air disinfection device comprising a UV-based disinfection zone.

### Background to the Invention

With forced ventilation present in shops, offices and new-build houses, it is known that spreading of viruses and bacteria is increasing. Places where lots of people gather or come close to one another, such as shopping malls, office centra or shared-offices, are known to be spaces where viruses and bacteria can infect people in mass, especially for viruses and bacteria that are transferred from human to human through the air or by small air droplets and aerosols present in air streams.

Air disinfection devices are known that are designed to remove virus particles and bacteria from the air, either by filtering the air, by destroying the genetic material from the viruses or bacteria through UV radiation or by a combination of both. In UV based air purifiers, air is forced through a pathway defined in a housing by means of a fan, whereby said pathway comprises a UV sterilisation chamber wherein the air flowing through is irradiated by a UV source.

As UV radiation is harmful to humans, is typically performed in the housing of an air purifier such that it is contained without the risk of harmful rays leaving the housing.

Known UV-based air disinfection devices however have the drawback of lacking efficiency and effectivity in that the air flow leaving the device often still contains viable viruses and or bacteria, especially when the load (degree of pollution/contamination of air) is high. In such conditions, when sterilisation of air is most important to humans, known devices fail to adequately remove sufficient virus particles and bacteria for sufficiently diminishing infection risks for people in the space where the air disinfection device is operating. Especially in view of the current COVID-19 crisis, there is an immediate need for effective and efficient air disinfection devices.

The current invention addresses the above drawback by providing a UV-based disinfection device allowing highly effective and efficient sterilisation of air streams, by a providing a specific air treatment comprising exposing the air flow to a UV radiation and by altering the air flow during the UV radiation or between two or more subsequent UV radiations within the device.

### Summary of the Invention

The present invention concerns an aerodynamic airflow disinfection device comprising a housing defining a pathway having an air input and an air output and at least a first UV-based disinfection zone and a second UV-based disinfection zone, both provided in serial configuration in the pathway between the air input and the air output, characterised in that at least a first and a second airstream modulator are provided in the pathway, the first airstream modulator arranged upstream the first disinfection zone and the second airstream modulator arranged between the first and second UV-based disinfection zones.

### Brief Description of the Figures

Fig. 1 schematically illustrates an air disinfection device according to the present invention;
Fig. 2 illustrates the air disinfection device of Fig. 1 from a viewpoint of arrow PI.

### Detailed Description

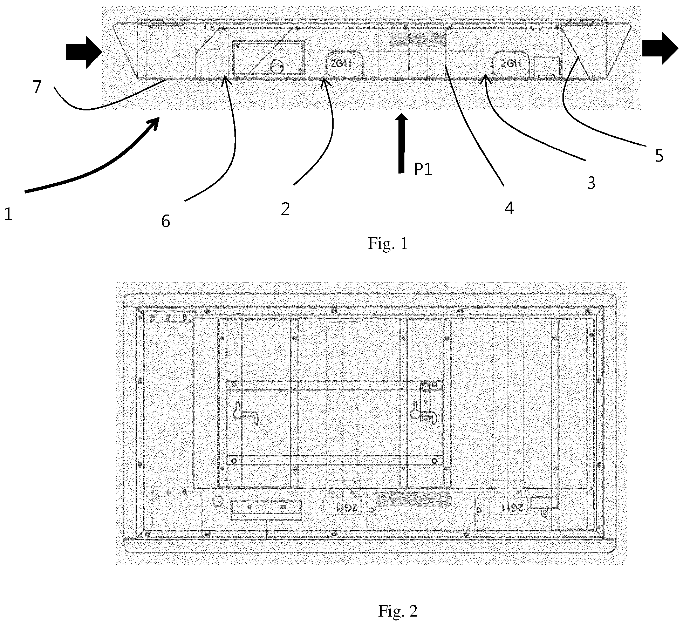

As illustrated in Fig. 1, a UV disinfection device according to the invention comprises a housing defining an internal pathway having an air input and an air output. pathway is preferably defined by a part of the housing made from a UVC and/or UVV resistant material such as aluminium.

At the input of the pathway, a fan is provided configured for drawing air into the pathway and creating a forced air stream through said pathway.

In accordance with the present invention, at least two UV-based disinfection zones are provided in the pathway together with at least two airstream modulators. The UV-based disinfection zones and airstream modulators are arranged in a serial configuration in the pathway, such that an airstream passing through the pathway from the input to the output consecutively passes the first airstream modulator, the first UV-based disinfection zone, the second airstream modulator and the second UV-based disinfection zone.

The UV-disinfection zones comprise one or more UV-emitting sources, emitting UV light having a wavelength capable of transversing cellular walls. The UV spectrum is divided into portions by wavelength. UVB and UVC radiation represents that portion of the spectrum that is capable of damaging biological organisms. High energy UVC photons are those with wavelengths shorter than 290 nm and are capable of traversing cellular walls. UVC radiation is used as a germicidal in order to kill airborne pathogens. UVB radiation is characterised by wavelengths between 290 and 320 nm and is also damaging to biological organisms. In order to kill pathogens, the UV radiation needs to be of a wavelength that can traverse the cellular walls. Studies have shown that the effective wavelengths for killing pathogens, such as bacteria, are in the 200 to 320 nm range. Still other studies indicate that wavelengths between 240 and 280 nm are most effective in killing a broad range of pathogens, with peak effectivity around 260 to 270 nm. The intensity or "flux" of the UV radiation is an important consideration in evaluating the effects of the UV radiation at the pathogenic level. The "UV radiation flux density" is related to the amount of radiation at the specified wavelength that reaches the surface of the pathogens. This UV radiation flux is also referred to as the "UV irradiance". In the interaction of radiant energy with biological organisms, both wavelength and irradiance, or radiation flux, must be considered. The "UV dosage" required to effectively kill airborne pathogens is derived from a combination of UV wavelength, radiation flux, and time of exposure. The "dwell" or "residence" time is defined as the amount of time that the airborne pathogens remain exposed to the UV radiation field and are irradiated by one or more UV LEDs. The desired pathogen kill rate can then be optimised by properly balancing the UV LED wavelength, radiation flux, and dwell time.

For adequate disinfection of the air, it is preferred that the dwell time is at least one second in case of irradiation with UV-light at a wavelength of between 200 and 320 nm.

The first and second airstream modulators each may comprise one or more vortex generating elements such as a series of baffles ordered in a parallel arrangement throughout the entire lateral cross section of the pathway or a grid having apertures having a cross-sectional area dimensioned to allow passing of air contaminents such as bacteria or dust particles, eg apertures of one or more square milimeters in cross-section. Alternatively, the one or more vortex generating elements may comprise rotating fan with a plurality of fins allowing compressing, twirling and varying the speed of the airstream passing therethrough, in which case, the fan for drawing air into the device may function as the first airstream modulator. The vortex-generating elements preferably have at least one wall generally extending in a direction of the air flow and in a transverse direction, perpendicular to the air flow with said at least one wall being shaped to impose on the flowing air a vorticity component.

The vortex generating element of the first airstream modulator preferably differs from the vortex generating element of the second airstream modulator in orientation in the pathway and/or in shape, such as to create a different (turbulent) airstream pattern in the first disinfection zone versus the second disinfection zone, in particular, it is preferred that the predominant vortexes created in the airstream in the first an second disinfection zones have a different orientation, most preferably have transversal to the airflow but at an angle differing 90° between the first and second disinfection zones. It is clear that in a device according to the present invention, each airstream modulator can itself comprise a series of vortex generating element or other airstream modulating elements such as laminar flow generating baffles or grids.

The air disinfection device according to the present invention preferably also comprises a third disinfection zone. According to a first embodiment, the third disinfection zone comprises a precipitator containing a row of thin vertical wires, and followed by a stack of large flat metal plates oriented vertically, with the plates typically spaced about 1 cm apart. The air stream flows horizontally through the spaces between the wires, and then passes through the stack of plates. A negative voltage of several thousand volts is applied between wire and plate. If the applied voltage is high enough, an electric corona discharge ionises the air around the electrodes, which then ionises the particles in the air stream. The ionised particles, due to the electrostatic force, are diverted towards the grounded plates. Particles build up on the collection plates and are removed from the air stream.

Alternatively, the third disinfection zone is embodied as a photo-ioniser applying lesser high-voltages as the above precipitator, but with the same goal of charging and subsequently removing larger particles such as bacteria from the airstream.

The third disinfection zone may be arranged in a serial configuration with both the first and second disinfection zone and either before the first and second disinfectin zones (ie upstream the first airstream modulator and upstream the first disinfection zone), between the first and second disinfection zone, in which case it is preferred to provide the third disinfection zone between the first disinfection zone and the second airstream modulator (ie downstream the first disinfection zone and upstream the second airstream modulator); or downstream the second disinfection zone.

With the above set-up of three distinct serially arranged disinfection zones, the need for a flow-through filter unit wherein particles are filtered out of the airstream on basis of the mesh size of the filter becomes redundant, rendering the device easier in maintenance and safer in use, as no potentially bacteria rich filter cake is created and the capacity of the device for disinfecting air does not alter due to particles trapped in the filter and limiting airflow therethrough. Furthermore, the lack of filter also provides the advantage that biological activity in the filter cake occurs, which often associated with unwanted odours.

It is clear that the device further comprises a control system allowing regulating the fan, the UV emitting sources and potentially the vortex creating elements to optimise the working of the air disinfection device of the invention in function of need, air quality, etc.

The device according to the present invention can be installed as stand-alone air disinfection devices in shopping malls, office centra, shared-offices, hospitals, shops, offices, houses, sport or other venues, etc. Alternatively, the device according to the invention can be integrated in a heating device, such as for example a far-infrared heating device used in shops, malls, offices, hospitals, venues, etc.
1 - airflow disinfection device
2, 3 - UV-based disinfection zones
4, 5 - airstream modulators
6 - photo-ionisation unit
7 - fan

## Claims

1. An air disinfection device comprising a housing defining a pathway having an air input and an air output and at least a first UV-based disinfection zone and a second UV-based disinfection zone, both provided in serial configuration in the pathway between the air input and the air output, **characterised in that** at least a first and a second airstream modulator are provided in the pathway, the first airstream modulator arranged upstream the first disinfection zone and the second airstream modulator arranged between the first and second UV-based disinfection zones.

2. The air disinfection device according to claim 1, said two airstream modulators configured to modulate the air stream according to a different pattern.

3. The air disinfection device according to claim 2, said different patterns being turbulent airstream patterns.

4. The air disinfection device according to any of the preceding claims, said airstream modulators comprising one or more vortex generating elements.

5. The air disinfection device according to any of the preceding claims, comprising a fan configured to create a forced airstream through the pathway.

6. The air disinfection device according to any of the preceding claims, comprising a third sterilisation zone, configured as a precipitator creating a high-voltage discharge there between, through which the airstream is guided.

7. The air disinfection device according to any of the preceding claims 1 to 5, comprising a third disinfection zone, wherein a photo-ionisation unit is provided.

8. The air disinfection device according to claims 6 or 7, wherein the third disinfection zone is arranged in the pathway serial to and in between the first and second disinfection zones.

9. The air disinfection device according to claim 8, wherein the third disinfection zone is arranged upstream the second airstream modulator.

10. The air disinfection device according to claims 6 or 7, wherein the third disinfection zone is arranged in the pathway serial to and downstream the second disinfection zone.

11. The air disinfection device according to claims 6 or 7, wherein the third disinfection zone is arranged in the pathway serial to and upstream the first and second disinfection zones.

12. The air disinfection device according to any of the preceding claims, not comprising a physical filter wherethrough the airstream is directed and accumulating airborne particles.

13. Method for disinfecting air, comprising subjecting an airstream to be disinfected to two UV radiation treatments and modulating said airstream between the first and second UV treatments thereby altering the pattern of the airflow between the first and second UV treatments.
